# EUROPEAN PATENT SPECIFICATION

(11) **EP 3 228 619 B1**
(45) Date of publication and mention of the grant of the patent: **29.07.2020**
(21) Application number: 16460019.9
(22) Date of filing: 04.04.2016
(51) Int. Cl.: C07D 471/04

(54) **PROCESS FOR THE PREPARATION OF APIXABAN**
VERFAHREN ZUR HERSTELLUNG VON APIXABAN
PROCÉDÉ DE PREPARATION DE APIXABAN

(43) Date of publication of application: 11.10.2017
(73) Proprietor: Zaklady Farmaceutyczne Polpharma SA, 83-200 Starogard Gdanski (PL)
(72) Inventor: Piotrkowska, Barbara, 14-520 Pieniezno (PL); Szulc, Marcin, 83-200 Starogard Gdanski (PL); Krzyzanowski, Mariusz, 83-220 Skórcz (PL)
(74) Representative: Chmurzynski, Sedzimir Lech

(56) References cited:
- WO-A1-2016/020711
- WO-A2-03/049681
- CN-A- 103 992 314
- CN-A- 104 045 637

## Description

The present invention relates to a process for the preparation of a pharmaceutical agent, and particularly to a process for preparing apixaban.

Apixaban is an anticoagulant used for the treatment of venous thromboembolism (VTE) and venous thromboembolic events. The medicament is often prescribed to address indications related to blood-clotting. For example, apixaban is used to lower the risk of stroke and embolism in patients displaying nonvalvular atrial fibrillation and in the prophylaxis of deep vein thrombosis and pulmonary embolism.

Apixaban is classified as a direct Factor Xa inhibitor. Factor Xa operates by cleaving prothrombin at two sites (arginine-threonine and arginine-isoleucine bonds), and these hydrolysis events yield active thrombin. Thrombin is an important component responsible for the clotting of blood, and therefore any reduction in its plasma concentration reduces the risk of an adverse blood-clotting event. The cleavage process is described as optimised when factor Xa is complexed with activated co-factor V in the prothrombinase complex, though both processes do also occur independently. Factor Xa is therefore essential to the downstream propagation of the coagulation cascade, through mediating the release of thrombin. Therefore, for patients at risk of experiencing an adverse blood-clotting event, inhibition of Factor Xa provides respite as a small molecule drug target.

Chemical inhibitors operate by blocking the active site of Factor Xa in both the blood plasma and the prothrombinase complex. Consequently, both the normal production of thrombin (through enzymatic cleavage of two sites on prothrombin) and thrombin levels are reduced. This reduction impacts upon the downstream production of fibrin and platelet activation, thus reducing the innate capacity of blood to clot.

Apixaban is named (1-(4-methoxyphenyl)-7-oxo-6-[4-(2-oxo-piperidin-1-yl)-phenyl]-4,5,6,7-tetrahydro-1*H*-pyrazolo[3,4-*c*]pyridine-3-carboxamide. The compound is represented structurally by Formula I.

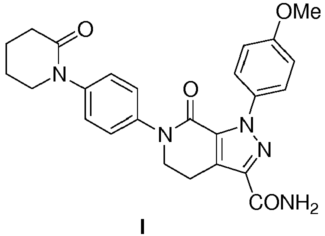

Apixaban (BMS-562247) is commercially marketed by Bristol-Myers Squibb and Pfizer under the trade name Eliquis®. The drug is formulated as an oral film-coated tablet in amounts of either 2.5 mg or 5 mg.

WO 03/049681 discloses a method of synthesis of apixaban, where the acidic precursor of apixaban is reacted with iso-butyl chloroformate and an amine delivering agent, resulting in a final yield of apixaban equal to 70%.

A further method is disclosed in WO 2016/020711 and CN104045637 where apixaban is obtained in a reaction of the acidic precursor of apixaban with carbonyldiimidazole and an amine.

Another method for obtaining apixaban is disclosed in CN103992314 and involves the use of tert-butyl chloroformate in the synthesis of apixaban with 70% yield.

US 7,371,761 discloses a process which involves a metal-catalysed cross coupling reaction between an aromatic iodide and 2-piperidinone, followed by ammonolysis to yield crude apixaban, as follows:

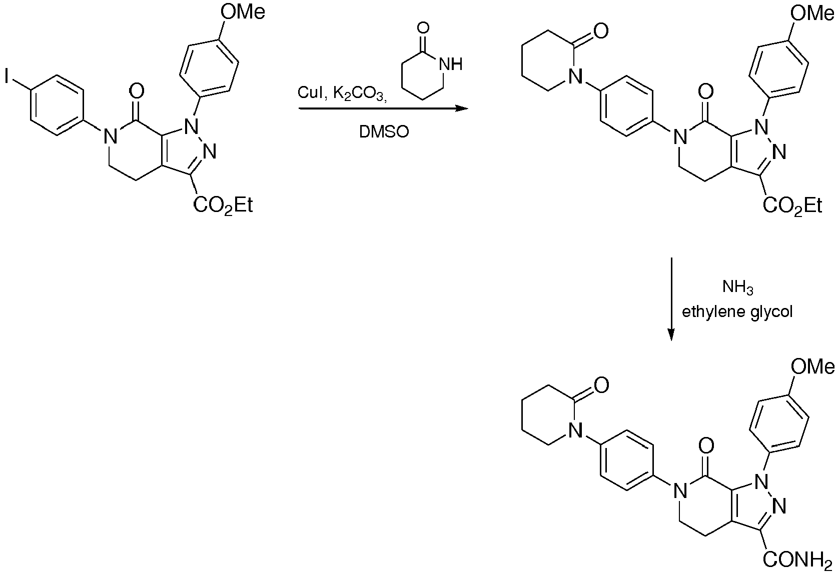

WO 2014/072884 discloses a synthesis of apixaban utilising a phenyl hydrazine derivative and an α-ketoester. The process is as follows:

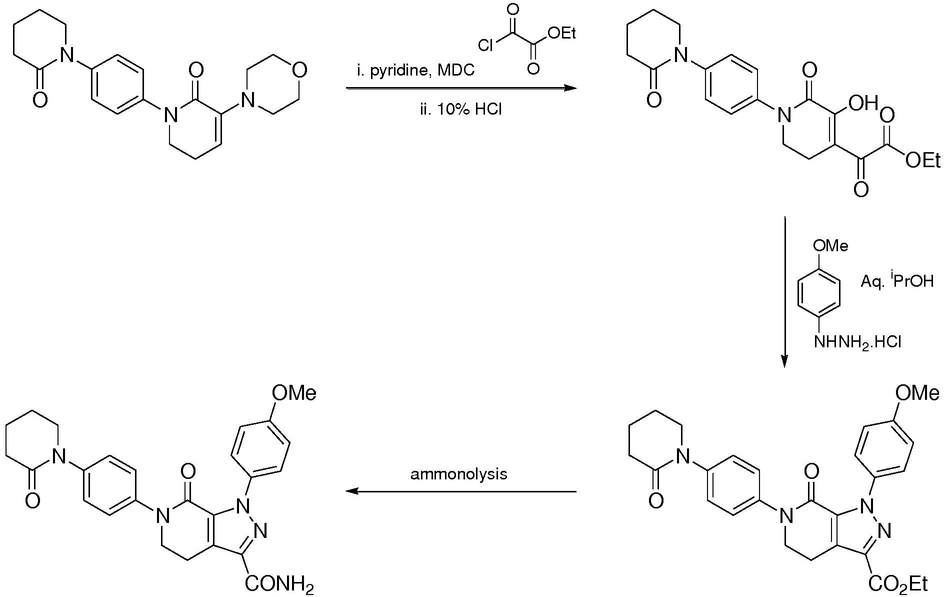

In US 7,371,761, WO 2014/072884 and various syntheses reported in the art, the terminating synthetic step relies upon the conversion of an ester to a primary amide (an ammonolysis reaction). The ammonolysis step is known to be capricious, and often apixaban is provided with a less than satisfactory yield and purity. Consequently, following conversion to the primary amide, the compound is subjected to multiple purification protocols. Typically, crude apixaban is purified first by column chromatography and second by a recrystallisation step. As with the ammonolysis step per se, the yield of material recovered from the recrystallisation step is also less than satisfactory. Such synthetic and procedural limitations are often fatal to the progression of a process from the laboratory to a large-scale chemical manufacturing plant.

The synthetic methodology employed to produce multi-kilogram quantities of a market-approved pharmaceutically active ingredient differ greatly from the methodologies employed at the discovery stage. Early stage drug discovery campaigns typically require the production of 5-10 mg of material (per molecule of interest). Such an amount allows for thorough characterisation of physical and chemical parameters, and moreover represents a workable quantity for use in biological assessment. Synthetic chemists operating at the discovery level generally do not assess whether a synthetic route taken to access a molecule of interest, is indeed scalable.

Large-scale chemical synthesis (or process chemistry) concerns the development, optimisation and production of multi-kilogram and multi-tonne quantities of pharmaceutically active ingredients and speciality bulk chemicals. Consequently, and owing to economies of scale, any small improvement in a given reaction or purification parameter is particularly economically significant. Therefore, optimisation of a large-scale synthesis that provides, for example, an increase in chemical yield, decrease in reaction time, decrease in reaction temperature, decrease in amount of catalyst or solvent used, increase in the favourability of reagents, reduction in side-product formation, a more environmentally benign synthesis or increase in chemical purity is of interest both to chemical manufacturers and suppliers. Moreover, step optimisation that reduces the need for multiple or hard-to-perform purifications are particularly beneficial. Conventional column chromatography and high-performance liquid chromatography (HPLC) are ubiquitous techniques, and resemble the cornerstone of the discovery chemist's purification arsenal. Unfortunately, these techniques are unsuitable for the production of multi-kilogram and multi-tonne quantities, yet begrudgingly, such purifications will have to be performed if the target is of high value and no feasible alternative exists. Consequently, any improvement in the ease of purification or isolation, through telescoping (wherein two or more, previously independent synthetic transformations converge into a "single" process and therefore require only one purification step) of synthetic procedures, or the identification of an intermediate for recrystallisation, or precipitation, or removal of impurities through conversion into a transient intermediate, or substitution for a cheaper, more environmentally friendly or less toxic reagent, provide an attractive and economically desirable goal. In situations where more traditional purification procedures yield poor or unsuitable results, it becomes necessary that more innovative solutions are required.

Practical achievement of any such goal is however not straightforward, and even careful optimisation of individual parameters of a synthetic or purification step will often fail to provide a workable advantage within an overall production process.

Although syntheses of apixaban are established, there remains a need in the art for improving the overall synthesis of apixaban, improving the purification of apixaban and increasing the purity of apixaban.

Accordingly, the present invention provides a process for preparing apixaban, comprising reacting a compound of formula II, 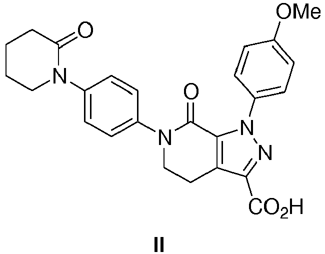 with cyanuric chloride as carboxylic acid activating agent and a source of ammonia.

The reaction of the present invention provides a transformation of a carboxylic acid moiety to a primary amide moiety. Such a reaction could be described as a functional group interconversion, and the reaction is depicted as the conversion of a compound of formula II to a compound of formula I (apixaban).

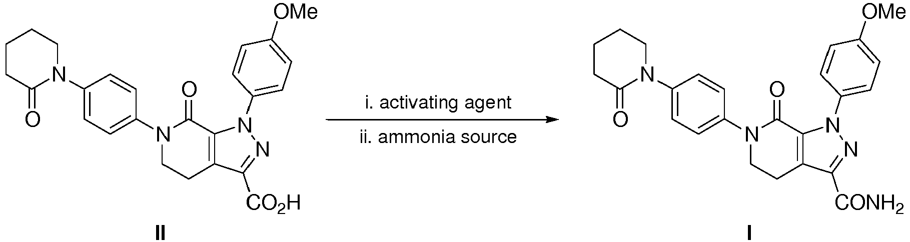

The use of carboxylic acids within chemical synthesis is widespread, as is their presence within molecules of biologic and pharmaceutical interest. However, from a synthetic viewpoint, carboxylic acids are inherently poor electrophiles, and hence are relatively inert and resistant to nucleophilic attack. The lack of electrophilicity of the carbonyl carbon atom is owing to the presence of the carboxylic acid -OH group directly bonded to it. Although electronegative per se, the oxygen atom is able to delocalise electron density through resonance forms, and hence the carbonyl carbon is electron-rich i.e. non-electrophilic.

In order to combat low reactivity, methods for increasing the electrophilicity of various functional groups have been developed within the art. Of all approaches for enhancing the electrophilicity of carboxylic acids, the use of activating agents is most prevalent, and many examples can be found in both automated and bench-based peptide chemistry. Such activating agents can be described as carboxylic acid activating agents or activating agents for carboxylic acids.

Activating agents can either co-ordinate to the carboxylic acid group, and deactivate the -OH group (by preventing resonance into the carbonyl carbon and hence increase the electropositivity at carbon) or the agents can replace the carboxylic acid -OH with a more reactive species, for example, by transforming a carboxylic acid into a more reactive acyl chloride or acyl imidazole species (i.e. an activated carboxylic acid synthon).

The term "synthon" as used in connection with the present invention is used to mean a chemical building block used to perform a synthetic operation.

Activating agents for carboxylic acids are known in the art and can be divided into several classes owing to their chemical composition, for example acyl chloride generating reagents (i.e. sources of nucleophilic chlorine (Cl⁻)), acid anhydrides, acyl imidazoles, acyliminocyanoacetates, *N*-substituted benzotriazoles (e.g. HOBt, HATU, BOP, PyBOP) and carbodiimides (e.g. DCC).

It is important to note, that in accordance with the present invention, reagents that are capable of transforming a carboxylic acid (e.g. the carboxylic acid contained within the compound of formula II) into an ester are not considered activating agents for carboxylic acids. Carboxylic acid activating agents per se react with carboxylic acids to provide an activated carboxylic acid synthon. Within the art, simple aliphatic (e.g. methyl, ethyl, propyl, isopropyl, butyl, *tert*-butyl) and simple aromatic (e.g. benzoyl) esters, although displaying additional electrophilic reactivity compared to carboxylic acids, are not considered activated carboxylic acid synthons. Therefore in accordance with the present invention it is considered that reagents used to conduct acid-catalysed esterification reactions (e.g. acid and an aliphatic or aromatic alcohol, e.g. hydrochloric acid and methanol) do not fall within the scope of the term "carboxylic acid activating agent".

Typical examples of carboxylic acid activating agents include, 1-hydroxybenzotriazole (HOBt), 1-hydroxy-7-azabenzotriazole (HOAt), 2-(1H-benzotriazol-1-yl)-1,1,3,3-tetramethyluronium hexafluorophosphate (HBTU), 1-[bis(dimethylamino)methylene]-1H-1,2,3-triazolo[4,5-b]pyridinium 3-oxid hexafluorophosphate (HATU), (benzotriazol-1-yloxy)tris(dimethylamino)phosphonium hexafluorophosphate (BOP), benzotriazol-1-yl-oxytripyrrolidinophosphonium hexafluorophosphate (PyBOP), and related benzotriazole derivatives, bromotripyrrolidinophosphonium hexafluorophosphate (PyBrop), bis(2-oxo-3-oxazolidinyl)phosphinic chloride (BOP-CI), diphenylphosphinic chloride (DPP-CI), pentafluorophenyl diphenylphosphinate (FDPP), N,N'-dicyclohexylcarbodiimide (DCC), 1-ethyl-3-(3-dimethylaminopropyl)carbodiimide (EDC), carbonyldiimidazole (CDI), N,N'-diisopropylcarbodiimide (DIC), 1,3-bis(2,2-dimethyl-1,3-dioxolan-4-ylmethyl)carbodiimide (BDDC), dimethylaminopyridine (DMAP), ethyl (hydroxyimino)cyanoacetate (Oxyma), (1-cyano-2-ethoxy-2-oxoethylidenaminooxy)dimethylamino-morpholino-carbenium hexafluorophosphate (COMU), oxalyl chloride, oxalyl chloride and catalytic DMF, thionyl chloride, thionyl chloride and catalytic DMF, phosphorous oxychloride, cyanuric chloride, phosgene, phosphorous trichloride, phosphorous pentachloride, triphenylphosphine and carbon tetrachloride, sodium azide and thiphenylphosphine or trichloroacetamide catalysts and diphenylphosphorylazide. Operating via similar chemical mechanisms, the formation of mixed anhydrides can also activate the carboxylic acid group, typically such methods involve the use of activating agents including chloroformate derivatives e.g. ethylchloroformate and benzyl chloroformate and quinoline derivatives e.g. *N*-ethoxycarbonyl-2-ethoxy-1,2-dihydroquinoline (EEDQ).

According to the present invention cyanuric chloride is used as activating agent.

Activation of carboxylic acids (i.e. the carboxylic acid contained within the compound of formula II) to form acyl chlorides can be performed in the presence or absence of a base.

The activating agent used in conjunction with the present invention typically provides apixaban in a yield greater than 80%, that is for the step of converting -CO₂H + activating agent, into -CONH₂.

In accordance with the present invention, cyanuric chloride is used as carboxylic acid activating agent in the presence of an alkyl amine-containing base. Typical alkyl amine-containing bases suitable for use with the present invention include, trimethylamine, triethylamine, tributylamine, triisobutylamine or diisopropylethylamine, piperidine, morpholine, *N*-methylmorpholine and piperazine. It is most preferred when *N*-methylmorpholine is an alkyl amine-containing base.

Following carboxylic acid activation, e.g. via the conversion of the carboxylic acid moiety into an acyl chloride moiety, the activating group must then undergo displacement by a nucleophile. In order to generate the primary amide present in the structure of apixaban, hence a nucleophile which can transfer an NH₂ group is required.

Sources of ammonia are able to transfer an NH₂ group as required by the present invention. Ammonia (NH₃) is a suitable nucleophile for addition to an activated carboxylic acid, e.g. an acyl chloride to thus generate a primary amide. Taking an acyl chloride as a particular example, a single molecule of ammonia can add into the acyl chloride carbonyl carbon atom and displace chlorine as a chloride anion. The resulting -NH₃⁺ group (now appended to the carbonyl) then undergoes a reaction with the previously displaced chloride anion. The chloride reacts with -NH₃⁺ to generate the desired primary amide appendage -NH₂ and a molecule of HCI.

In accordance with the present invention, suitable sources of ammonia include ammonium hydroxide and gaseous ammonia.

Ammonium hydroxide is also known as ammonia solution, ammonia water, ammonical liquor, ammonia liquor, aqua ammonia, aqueous ammonia and is often denoted by NH_{3(aq)} in chemical nomenclature. The name ammonium hydroxide suggests an alkali composition comprising [NH₄⁺][OH⁻], however it is not possible to isolate a sample of NH₄OH because the ions do not comprise a significant fraction of the total amount of ammonia in water. More precisely, a 1 molar solution of ammonia contains approximately 0.4% NH₄OH (ammonium), the rest being present as NH_{3(aq)}.

Gaseous ammonia is also suitable for use in accordance with the present invention. Typically following the reaction of a compound of formula II with a carboxylic acid activating agent, gaseous ammonia can be bubbled into the reaction solution in order to displace or react with the activated carboxylic acid, to generate the desired primary amide.

Aside from the use of molecular ammonia, other reagents exist in the art that are capable of delivering an -NH₂ group upon reaction with an activated carboxylic acid derivative. Depending on downstream transformations and existing functionalities within a molecule of interest, protected (NH₂) synthons can also be used to react with activated carboxylic acids. For example, benzylamine can be used, and following the addition reaction, the benzyl group can be cleaved with hydrogen to expose a primary amide.

In accordance with the present invention, it is preferred when the source of ammonia is ammonium hydroxide.

In accordance with the present invention, the process for preparing apixaban, comprising reacting a compound of formula II with cyanuric chloride as carboxylic acid activating agent and a source of ammonia, is preferably conducted in a solvent (i.e. an aqueous or organic solvent) or a mixture of solvents.

It is known in the art which particular solvent is likely to be most appropriate and most effective for each category of a reactive chemical transformation. For example, water- or air-sensitive transformations require the use of non-aqueous, non-protic solvents such as acetonitrile, tetrahydrofuran, dioxane, dimethylsulfoxide dichloromethane, chloroform or *N*-methylpyrrolidine. Furthermore, water- or air-sensitive reactions may also require an atmosphere of inert gas (to prevent atmospheric water from compromising a transformation) typical inert gases include nitrogen and argon. Alternatively, reactions requiring the use of an aqueous reagent (e.g. aqueous hydrochloric acid or aqueous sodium hydroxide) may require the use of one or more organic co-solvents. In this context, an organic co-solvent is added to aid the dissolution, or partial dissolution of a less polar organic reagent. Typical organic co-solvents for use with such aqueous reagents include, toluene, alcohols (methanol, ethanol, propanol), tetrahydrofuran and dimethylsulfoxide. Depending upon the particular process, the only limitation in regard to reaction efficacy when using organic co-solvents with aqueous solvents is the requirement for some degree of miscibility of the organic co-solvent with water. To aid problems with miscibility and or reaction efficacy a phase transfer catalyst may be employed (e.g. tetrabutylammonium bromide).

In accordance with the present invention, it is preferred wherein the process for preparing apixaban, comprising reacting a compound of formula II with cyanuric chloride as carboxylic acid activating agent and a source of ammonia, is conducted in a halogenated solvent. The most preferred halogenated solvent is dichloromethane.

Advantageously, the process for preparing apixaban, comprising reacting a compound of formula II with cyanuric chloride as carboxylic acid activating agent and a source of ammonia, provides apixaban with high purity. That is, high purity apixaban is provided in a single step obviating the need for performing time consuming chromatographic techniques, which reduce chemical yields.

Preferably, the process for preparing apixaban, comprising reacting a compound of formula II with cyanuric chloride as carboxylic acid activating agent and a source of ammonia, is conducted wherein 2 to 120 molar equivalents of an ammonia source are used with respect to 1 molar equivalent of a compound of formula II. More preferably, 20 to 100 molar equivalents of an ammonia source are used with respect to 1 molar equivalent of a compound of formula II.

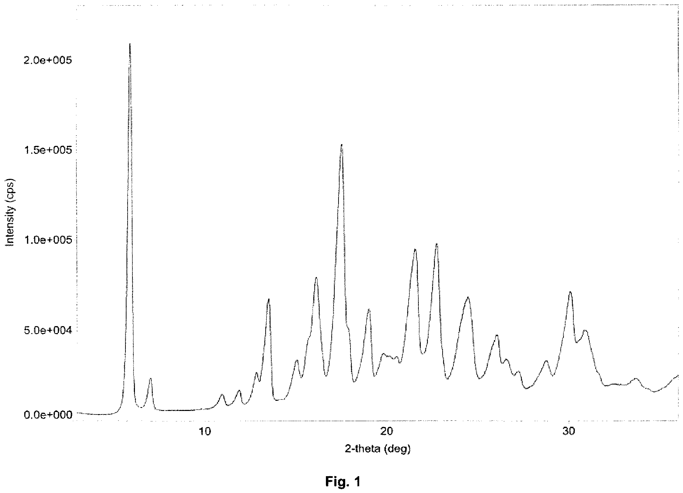

In accordance with the present invention, the amount of an ammonia source used (with respect to a compound of formula II) provides sufficient ammonia to transform an activated carboxylic acid derivative to a primary amide derivative whilst concomitantly providing the primary amide with a level of high purity. Furthermore, according to the present invention, the process for preparing apixaban, comprising reacting a compound of formula II with cyanuric chloride as carboxylic acid activating agent and ammonium hydroxide, is preferably conducted wherein the amount of ammonium hydroxide used is sufficient to transform an activated carboxylic acid derivative to a primary amide derivative whilst concomitantly providing apixaban with a level of high purity and as the hydrate polymorph of apixaban (the XRPD of apixaban hydrate is provided by Fig. 1).

Preferably, the hydrate polymorph of apixaban is provided with a level of high purity, wherein the process of the present invention is conducted with ammonium hydroxide as the source of ammonia. Advantageously, the use of an ammonia source in the process of the present invention provides apixaban with a high level of purity.

Upon reaction of a compound of formula II with cyanuric chloride as carboxylic acid activating agent, it is possible that full conversion to the activated carboxylic acid synthon derivative is not realised. In such circumstances, unreacted carboxylic acid will be present during the nucleophilic reaction with a source of ammonia. The presence of unreacted carboxylic acid is of no detriment to the reaction's efficacy. The carboxylic acid present within any (unreacted) compound of formula II will be deprotonated (CO₂H to CO₂⁻) upon addition of the source of ammonia. The deprotonation event alters the solubility profile of the unreacted reagent, and therefore during the reaction work up procedure, all unreacted (and consequently deprotonated) compound of formula II will reside in aqueous solvent. Extraction of the primary amide product into an organic solvent provides apixaban with a high level of purity, owing to the lack solubility of any unreacted material in organic solvent.

Therefore, in accordance with the present invention, there is also provided a process for preparing apixaban, comprising reacting a compound of formula II, 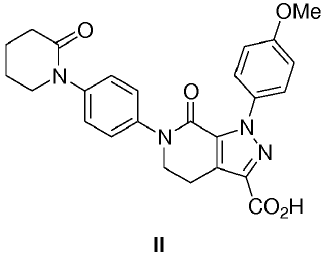 with cyanuric chloride as carboxylic acid activating agent and a source of ammonia, wherein apixaban is concomitantly purified by the source of ammonia.

It is therefore most preferred that the processes of the present invention further comprise a purification step, wherein apixaban is purified with a source of ammonia. Most preferably the source of ammonia is ammonium hydroxide. Following the purification step as described, the hydrate polymorph of apixaban is provided with a level of high purity.

It is also preferred that the process of the present invention, comprising reacting a compound of formula II, with cyanuric chloride as carboxylic acid activating agent and a source of ammonia, further comprises a purification step, wherein the agent used for the purification step is sodium hydroxide. Following the purification step as described, the hydrate polymorph of apixaban is provided with a level of high purity.

Advantageously, the processes of the present invention provide apixaban with a level of purity such that column chromatography is unnecessary.

Preferably, the process for preparing apixaban, comprising reacting a compound of formula II with cyanuric chloride as carboxylic acid activating agent and ammonium hydroxide, is conducted wherein 2 to 120 molar equivalents of ammonium hydroxide are used with respect to 1 molar equivalent of a compound of formula II. More preferably, 20 to 100 molar equivalents of ammonium hydroxide are used with respect to 1 molar equivalent of a compound of formula II.

Preferably, the process for preparing apixaban, comprising reacting a compound of formula II with cyanuric chloride as carboxylic acid activating agent and a source of ammonia, is conducted wherein a molar excess of a source of ammonia is used with respect to a compound of formula II.

Most preferably, the process for preparing apixaban, comprising reacting a compound of formula II with cyanuric chloride and ammonium hydroxide, is conducted wherein a molar excess of ammonium hydroxide is used with respect to a compound of formula II.

Preferably, the process for preparing apixaban, comprising reacting a compound of formula II with cyanuric chloride as carboxylic acid activating agent and ammonium hydroxide, is conducted wherein 2 to 120 molar equivalents of ammonium hydroxide are used with respect to 1 molar equivalent of a compound of formula II. More preferably, 20 to 100 molar equivalents of ammonium hydroxide are used with respect to 1 molar equivalent of a compound of formula II.

According to the present invention, the chemical purity of apixaban and intermediates thereof can be performed and measured by UPLC (ultra-performance liquid chromatography). Preferred solvents for analysing the purity of apixaban by ultra-performance liquid chromatography (UPLC) are ammonium bicarbonate buffer (5 mM) pH 8.6 in H₂O and acetonitrile. UPLC analysis can be performed, for example using a 100 x 2.1 mm Waters Acquity C18 RP column, where detection is set at between 200-400nm. Purity data as provided by the examples was acquired through use of the techniques mentioned hereinabove.

In accordance with the present invention, apixaban is obtainable by any one of processes described hereinabove, more preferably apixaban with high purity is obtainable by any one of processes described hereinabove, even more preferably apixaban with a purity greater than 99.95% (as measured by UPLC) is obtainable by any one of processes described hereinabove.

Preferably, the process for preparing apixaban, comprising reacting a compound of formula II with cyanuric chloride as carboxylic acid activating agent and a source of ammonia provides apixaban with high purity. That is, high purity apixaban is provided in a single step obviating the need for performing time consuming chromatographic techniques, and which often cause reduced chemical yields.

More preferably, the process for preparing apixaban, comprising reacting a compound of formula II with cyanuric chloride as carboxylic acid activating agent and ammonium hydroxide, is conducted wherein 2 to 120 molar equivalents of ammonium hydroxide are used with respect to 1 molar equivalent of a compound of formula II, and the process provides apixaban with high purity. More preferably, 20 to 100 molar equivalents of ammonium hydroxide are used with respect to 1 molar equivalent of a compound of formula II, and the process provides apixaban with high purity.

The purity of apixaban can also be increased by performing a recrystallisation step.

In accordance with the present invention, the process for the production of apixaban can additionally comprise a recrystallisation step. Recrystallisation provides a favourable method for purification, particularly favourable when conducted in connection with a large-scale industrial process. The process takes advantage of the differing solubilities of compounds. In essence, through dissolving both impurities and target compounds in an appropriate solvent, either the target compound or impurities can be selectively crystallised out of solution. A simple filtration and drying step allows for the collection of the target compound, circumventing the need for more elaborate, time consuming and expensive purification techniques.

The process of the present invention, despite providing apixaban with high a level of purity, following reacting a compound of formula II with cyanuric chloride as carboxylic acid activating agent and a source of ammonia, or following reacting a compound of formula II with cyanuric chloride as carboxylic acid activating agent and a source of ammonia wherein 2 to 120 molar equivalents of a source of ammonia are used with respect to 1 molar equivalent of a compound of formula II, or following reacting a compound of formula II with cyanuric chloride as carboxylic acid activating agent and ammonium hydroxide wherein 2 to 120 molar equivalents of ammonium hydroxide are used with respect to 1 molar equivalent of a compound of formula II, may additionally comprise a recrystallisation step.

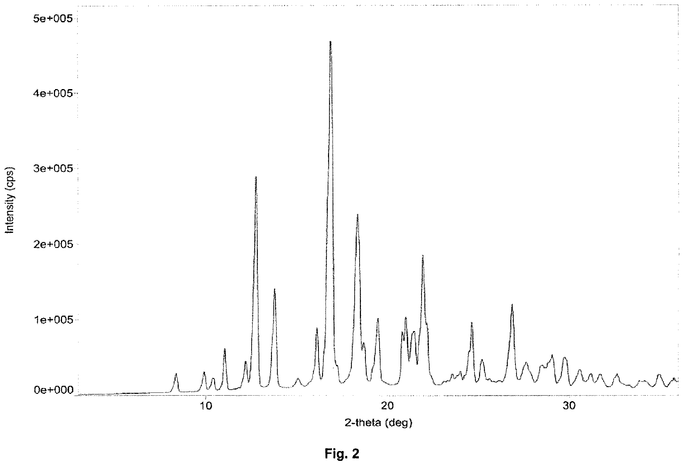

Typically, apixaban is recrystallised from an alcoholic solvent, alcoholic solvents include methanol, ethanol, propanol and isopropyl alcohol. Isopropyl alcohol is a particularly preferred solvent for the recrystallisation of apixaban. Apixaban is also preferably recrystallised from a mixture of isopropyl alcohol and water, particularly isopropyl alcohol and deionised water. According to the present invention, apixaban recrystallised by isopropyl alcohol alone, or isopropyl alcohol and deionised water is provided as a crystalline solid, preferably in crystalline anhydrous polymorphic form (the XRPD of the anhydrous apixaban is provided by Fig. 2).

Apixaban obtained by the processes of the present invention, is preferably recrystallised using isopropyl alcohol. It is also preferred when apixaban obtained by the processes of the present invention, is recrystallised using a mixture of isopropyl alcohol and deionised water. According to the present invention, apixaban recrystallised by isopropyl alcohol alone, or isopropyl alcohol and deionised water is provided as a crystalline solid, preferably in crystalline anhydrous polymorphic form.

To perform a recrystallisation step apixaban is typically suspended in one or more preferred solvents, and the suspension is heated, for example to reflux temperature in order to facilitate dissolution. Upon dissolution, solutions are then cooled to allow crystal formation.

The recrystallisation steps typically provide apixaban with a bulk purity of at least 99.5% as measured by UPLC.

The term "bulk" should be understood as applying to amounts of apixaban that weigh 1 kilogram or more, that is amounts that are to be produced at a process chemistry level (i.e. an industrial plant scale process).

It can be seen that the present invention provides a process for preparing apixaban, comprising reacting a compound of formula II, with cyanuric chloride as carboxylic acid activating agent and a source of ammonia.

The present invention will now be described with reference to the examples, which are not intended to be limiting.

### Examples

### Example 1

### 1-(4-Methoxyphenyl)-7-oxo-6-[4-(2-oxo-piperidin-1-yl)-phenyl]-4,5,6,7-tetrahydro-1 H-pyrazolo[3,4-c]pyridine-3-carboxylic acid

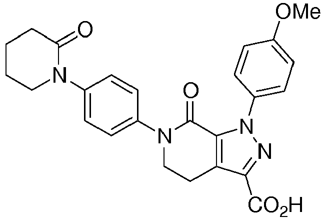

THF (1,000 mL) was loaded into a reaction vessel and ethyl 1-(4-methoxyphenyl)-7-oxo-6-[4-(2-oxo-piperidin-1-yl)-phenyl]-4,5,6,7-tetrahydro-1H-pyrazolo[3,4-c]pyridine-3-carboxylate (100 g) was added at ambient temperature (20-25°C). EtOH (500 mL) and aq. NaOH solution (16 g NaOH in 2000 mL of water) were added to the suspension. The mixture was stirred for 3 hrs at 20-25°C. After which, 1N HCl (2000 mL) was added dropwise at 20-30°C over a period of 30 minutes. During the addition of HCl product precipitation occurred. After complete addition the reaction mixture was stirred for 2 hrs at 20-25°C and the product was filtered. The filter cake was washed with water (2 x 500 mL) until the pH of the washing liquors was above pH 4. The product was dried at 85-90°C for 16 hrs, after which 89.7 g was obtained (95% yield) and UPLC purity 99.8%.

### Example 2

### 1-(4-Methoxyphenyl)-7-oxo-6-[4-(2-oxo-piperidin-1-yl)-phenyl]-4,5,6,7-tetrahydro-1H-pyrazolo[3,4-c]pyridine-3-carboxamide (apixaban)

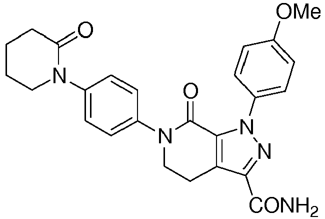

Dichloromethane (2000 mL) was loaded into a reaction vessel and 1-(4-methoxyphenyl)-7-oxo-6-[4-(2-oxo-piperidin-1-yl)-phenyl]-4,5,6,7-tetrahydro-1*H*-pyrazolo[3,4-*c*]pyridine-3-carboxylic acid (50 g) was added at ambient temperature (20-25°C). 4-Methylmorpholine (12.75 mL) was added and the mixture was stirred for 15 minutes (until complete dissolution of the solid). Cyanuric chloride (21 g) was added and the mixture was stirred for 1 hr at 20-25°C. Then, aqueous ammonia solution (25%, 250 mL) was added and the mixture was stirred vigorously for 3 hrs at 20-25°C. After which, a 5% NaOH aqueous solution (750 mL) was added to the reaction vessel, and stirring was continued for 20 minutes. After phase separation the organic layer was washed with 5% NaOH (375 mL), 1 N HCl (300 mL) and water (1000 mL). Next, ethyl acetate (2000 mL) was added dropwise to the organic layer over 1.5-2 hr. After complete addition the mixture was stirred for 2 hrs at 20-25°C. The product was collected via filtration, washed with an ethyl acetate : DCM mixture (1:1, v/v, 200 mL) and dried at 85-90°C for 16 hrs, after which 44.0 g was obtained of apixaban hydrate (82% yield) and UPLC purity 99.2%, water content 7.0% (KF).

### Example 3

### 1-(4-Methoxyphenyl)-7-oxo-6-[4-(2-oxo-piperidin-1-yl)-phenyl]-4,5,6,7-tetrahydro-1H-pyrazolo[3,4-c]pyridine-3-carboxamide (apixaban)

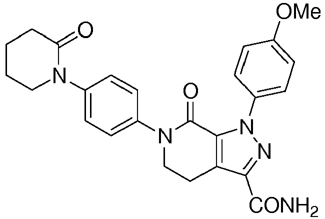

Dichloromethane (400 mL) was loaded into a reaction vessel and 1-(4-methoxyphenyl)-7-oxo-6-[4-(2-oxo-piperidin-1-yl)-phenyl]-4,5,6,7-tetrahydro-1*H*-pyrazolo[3,4-*c*]pyridine-3-carboxylic acid (10 g) was added at ambient temperature (20-25°C). 4-Methylmorpholine (2.56 mL) was added and the mixture was stirred for 15 minutes (until complete dissolution of the solid). Cyanuric chloride (4.25 g) was added and the mixture was stirred for 1 hr at 20-25°C. Then, aqueous ammonia solution (25%, 50 mL) was added and the mixture was stirred vigorously for 3 hrs at (20-25°C). After which, water (150 mL) was added, followed by aqueous ammonia solution (25%, 100 mL) and stirring was continued for 15 min. After phase separation the organic layer was washed with mixtures of water (50 mL) and aqueous ammonia solution (25%, 50 mL), and 1 N HCl (60 mL) and water (200 mL). Next, ethyl acetate (400 mL) was added dropwise to the organic layer over 10 min. After complete addition the mixture was stirred for 2 hrs at 20-25°C. The product was filtered and dried at 85-90°C for 16 hrs, after which 8.9 g was obtained of apixaban hydrate (83% yield) and UPLC purity 99.2%, water content 6.9% (KF).

### Example 4

### 1-(4-Methoxyphenyl)-7-oxo-6-[4-(2-oxo-piperidin-1-yl)-phenyl]-4,5,6,7-tetrahydro-1H-pyrazolo[3,4-c]pyridine-3-carboxamide (apixaban)

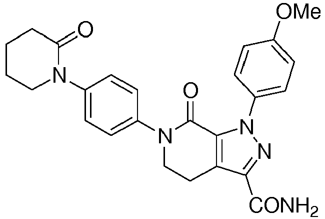

Dichloromethane (400 mL) was loaded into a reaction vessel and 1-(4-methoxyphenyl)-7-oxo-6-[4-(2-oxo-piperidin-1-yl)-phenyl]-4,5,6,7-tetrahydro-1*H*-pyrazolo[3,4-*c*]pyridine-3-carboxylic acid (10 g) was added at ambient temperature (20-25°C). 4-Methylmorpholine (2.56 mL) was added and the mixture was stirred (until complete dissolution of the solid). Cyanuric chloride (4.25 g) was added and the mixture was stirred for 1 hr at 20-25°C. Then, aqueous ammonia solution (25%, 150 mL) was added and the mixture was stirred vigorously for 3 hrs at (20-25°C). Next, the phases were separated and the organic layer was washed with mixture of water (50 mL) and aqueous ammonia solution (25%, 50 mL), 1 N HCl (60 mL) and water (200 mL). Ethyl acetate (400 mL) was added dropwise to the organic layer over 10 min. After complete addition the mixture was stirred for 2 hrs at 20-25°C. The product was filtered and dried at 85-90°C for 16 hrs, after which 8.9 g was obtained of apixaban hydrate (83% yield) and UPLC purity 99.1%, water content 7.2% (KF).

### Example 5

### Recrystallisation of 1-(4-Methoxyphenyl)-7-oxo-6-[4-(2-oxo-piperidin-1-yl)-phenyl]-4,5,6,7-tetrahydro-1H-pyrazolo[3,4-c]pyridine-3-carboxamide (apixaban)

Deionised water (100 mL) was loaded into a reaction vessel and apixaban hydrate (30 g) was added thereto at ambient temperature (20-25°C). Isopropyl alcohol (300 mL) was added and the mixture was heated to reflux temperature and maintained for 30 minutes (until complete dissolution). Activated carbon (2 g) was then added, and stirring at reflux was continued for a further 30 minutes. The charcoal was removed via filtration (hot filtration 80-90°C) and the filtrate was cooled to 20-25°C over a period of 5.5-6.5 hrs (at 70-75°C seeding). The mixture was stirred at 20-25°C for 4 hrs and the crystallised product was isolated via filtration. The product was then dried at 85-90°C for 16 hrs, after which 24 g was obtained of anhydrous apixaban (80.0% yield) and UPLC purity 99.95%, water content 0.19 % (KF).

### Example 6

### Recrystallisation of 1-(4-Methoxyphenyl)-7-oxo-6-[4-(2-oxo-piperidin-1-yl)-phenyl]-4,5,6,7-tetrahydro-1H-pyrazolo[3,4-c]pyridine-3-carboxamide (apixaban)

Isopropanol (20 L) was loaded into a reaction vessel and the hydrate of 1-(4-methoxyphenyl)-7-oxo-6-[4-(2-oxo-piperidin-1-yl)-phenyl]-4,5,6,7-tetrahydro-1*H*-pyrazolo[3,4-c]pyridine-3-carboxamide (100 g) was added. The mixture was heated to reflux (∼82°C) with stirring until dissolution occurred. The reaction mixture was then cooled to 0-5°C and the temperature maintained for 3 hours. The precipitated product was removed by filtration and the product was washed with isopropanol (2 L). The resulting solid was dried in an air drier (at 90°C) and recrystallised anhydrous apixaban was obtained 92.9 g (93% yield) and UPLC purity 99.89%, water content 0.16% (KF).

### Comparative Example 1

### 1-(4-Methoxyphenyl)-7-oxo-6-[4-(2-oxo-piperidin-1-yl)-phenyl]-4,5,6,7-tetrahydro-1H-pyrazolo[3,4-c]pyridine-3-carboxamide (apixaban)

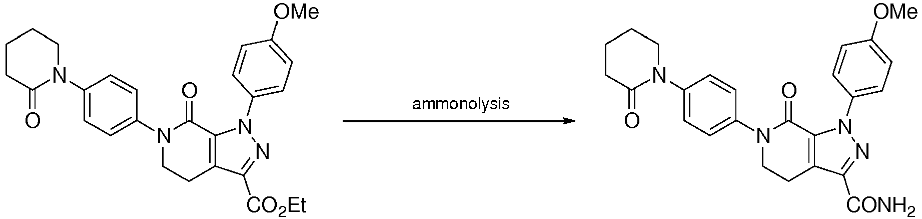

To ethyl-1-(4-methoxyphenyl)-7-oxo-6-[4-(2;oxo-piperidin-1-yl)-phenyl]-4,5,6,7-tetrahydro-1*H-*pyrazolo[3,4-c]pyridine-3-carboxylate (4.8 g, 9 mmol) was added 5% NH₃ in ethylene glycol (40 mL) and the mixture was heated to 120°C for 4 h in sealed vessel. Water was added and the resulting solid was collected. Purification by silica gel chromatography using 0-10% MeOH/CHCl₃ as eluent afforded 3.5 g of a white solid. A portion of the solid was recrystallized from CH₂Cl₂/ EtOAc to afford 2.5 g of the title compound. The remaining solid and filtrate material was recrystallized from isopropyl alcohol to afford an additional 0.57 g for a total of 3.07 g (68% yield): ¹H NMR (CDCl₃) δ 7.49 (d, J = 8.8 Hz, 2H), 7.37 (d, J = 9.1 Hz, 2H), 7.26 (d, J = 8.8 Hz, 2H), 6.98 (s, 1H) 6.95 (d, J = 9.2 Hz, 2H), 6.28 (s,1H), 4.14 (t, J = 6.6 Hz, 2H), 3.81 (s, 3H), 3.61 (m, 2H), 3.39 (t, J = 6.6 Hz, 2H), 2.63 (t, J = 6.2 Hz, 2H), 1.96 (m, 4H).

## Claims

1. A process for the preparation of apixaban, comprising reacting a compound of formula II, 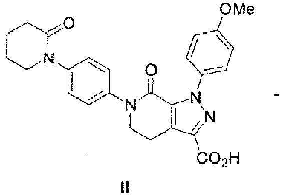 with cyanuric chloride and a source of ammonia.

2. A process as claimed in claim 1, wherein the source of ammonia is ammonium hydroxide.

3. A process as claimed in claim 2, wherein 2 to 120 molar equivalents of ammonium hydroxide are used with respect to the compound of formula II.

4. A process as claimed in claim 3, wherein 20 to 100 molar equivalents of ammonium hydroxide are used with respect to the compound of formula II.

5. A process as claimed in any one of the preceding claims, further comprising a purification step, wherein apixaban is purified with a source of ammonia, ammonium hydroxide or sodium hydroxide, to provide apixaban hydrate.

6. A process as claimed in any one of the preceding claims, wherein apixaban is provided with a purity of >99.0 % as measured by UPLC.

7. A process as claimed in any one of the preceding claims further comprising a recrystallisation step.

8. A process as claimed in claim 7 , wherein the solvent for recrystallisation comprises isopropyl alcohol or a mixture of isopropyl alcohol and water.

9. A process as claimed in claim 8 , wherein the polymorphic structure of the recrystallised apixaban is the anhydrous polymorphic form.

10. A process as claimed in claims 8 or 9 , wherein recrystallised apixaban has a bulk purity of at least 99.5% as measured by UPLC.

11. A process as claimed in any one of the preceding claims further comprising an alkyl amine-containing base.

## Patentansprüche

1. Verfahren zur Herstellung von Apixaban, das die Umsetzung der Verbindung der Formel II 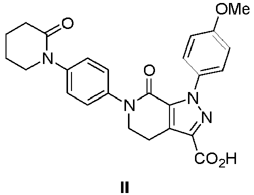 mit Cyanurchlorid und einer Ammoniakquelle umfasst.

2. Verfahren nach Anspruch 1, wobei Ammoniumhydroxid die Quelle von Ammoniak ist.

3. Verfahren nach Anspruch 2, wobei 2 bis 120 Moläquivalente von Ammoniumhydroxid bezogen auf die Verbindung der Formel II verwendet werden.

4. Verfahren nach Anspruch 3, wobei 20 bis 100 Moläquivalente von Ammoniumhydroxid bezogen auf eine Verbindung der Formel II verwendet werden.

5. Verfahren nach einem der vorstehenden Ansprüche, das ferner einen Reinigungsschritt umfasst, bei dem Apixaban mit einer Quelle von Ammoniak, Ammoniumhydroxid oder Natriumhydroxid gereinigt wird, um Apixaban-Hydrat bereitzustellen.

6. Verfahren nach einem der vorstehenden Ansprüche, wobei Apixaban mit einem Reinheitsgrad von > 99,0%, gemessen mit Hilfe von UPLC, bereitgestellt wird.

7. Verfahren nach einem der vorstehenden Ansprüche, das ferner einen Rekristallisationsschritt umfasst.

8. Verfahren nach Anspruch 7, wobei das Lösungsmittel für die Rekristallisation Isopropylalkohol oder ein Gemisch aus Isopropylalkohol und Wasser umfasst.

9. Verfahren nach Anspruch 8, wobei die polymorphe Struktur von rekristallisiertem Apixaban die wasserfreie polymorphe Form ist.

10. Verfahren nach Anspruch 8 oder 9, wobei rekristallisiertes Apixaban einen Reinheitgrad der Bulkware von mindestens 99,5% hat, gemessen mit Hilfe von UPLC.

11. Verfahren nach einem der vorstehenden Ansprüche, das ferner eine alkylaminhaltige Base umfasst.

## Revendications

1. Procédé pour la préparation de l'apixaban, comprenant la réaction d'un composé de formule II, 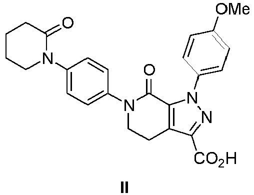 avec du chlorure cyanurique et une source d'ammoniac.

2. Procédé selon la revendication 1 dans lequel la source d'ammoniac est de l'hydroxyde d'ammonium.

3. Procédé selon la revendication 2 dans lequel 2 à 120 équivalents molaires d'hydroxyde d'ammonium sont utilisés par rapport au composé de formule II.

4. Procédé selon la revendication 3 dans lequel 20 à 100 équivalents molaires d'hydroxyde d'ammonium sont utilisés par rapport au composé de formule II.

5. Procédé selon l'une quelconque des revendications précédentes comprenant par ailleurs une étape de purification, où l'apixaban est purifié avec une source d'ammoniac, d'hydroxyde d'ammonium ou d'hydroxyde de sodium, pour fournir de l'hydrate d'apixaban.

6. Procédé selon l'une quelconque des revendications précédentes dans lequel l'apixaban est fourni à une pureté supérieure à 99,0% telle que mesurée par UPLC.

7. Procédé selon l'une quelconque des revendications précédentes comprenant par ailleurs une étape de recristallisation.

8. Procédé selon la revendication 7 dans lequel le solvant pour la recristallisation comprend de l'alcool isopropylique ou un mélange d'alcool isopropylique et d'eau.

9. Procédé selon la revendication 8 dans lequel la structure polymorphe de l'apixaban recristallisé est la forme polymorphe anhydre.

10. Procédé selon la revendication 8 ou 9 dans lequel l'apixaban recristallisé a une pureté en vrac supérieure ou égale à 99,5% telle que mesurée par UPLC.

11. Procédé selon l'une quelconque des revendications précédentes comprenant par ailleurs une base contenant des alkylamines.
